# EUROPEAN PATENT SPECIFICATION

(11) **EP 3 193 760 B1**
(45) Date of publication and mention of the grant of the patent: **03.11.2021**
(21) Application number: 14902101.6
(22) Date of filing: 15.09.2014
(51) Int. Cl.: A61B 18/12, A61B 17/32, A61B 18/00, A61B 18/14

(54) **SURGICAL SYSTEM HAVING DETACHABLE COMPONENT AND STATE DETECTION CIRCUIT FOR DETECTION OF STATE OF ATTACHMENT OF DETACHABLE COMPONENT**
CHIRURGISCHES SYSTEM MIT ABNEHMBARER KOMPONENTE UND ZUSTANDSERFASSUNGSSCHALTUNG ZUR ERFASSUNG DES ZUSTANDS DER BEFESTIGUNG DER ABNEHMBAREN KOMPONENTE
SYSTÈME CHIRURGICAL COMPRENANT UN ÉLÉMENT AMOVIBLE ET UN CIRCUIT DE DÉTECTION D'ÉTAT POUR LA DÉTECTION D'ÉTAT DE FIXATION D'ÉLÉMENT AMOVIBLE

(43) Date of publication of application: 26.07.2017
(60) Divisional application: 21192712.4
(73) Proprietor: Gyrus ACMI, Inc., d.b.a. Olympus Surgical Technologies America, Southborough, MA 01772 (US)
(72) Inventor: CHURCH, David C., Millington, Tennessee 38053 (US); BLUVSHTEIN, Vlad, Plymouth, Minnesota 55442 (US); LUCKE, Lori E., Rosemount, Minnesota 55068 (US)
(74) Representative: Seemann & Partner Patentanwälte mbB
(86) International application number: PCT/US2014/055611
(87) International publication number: WO 2016/043696

(56) References cited:
- EP-A2- 2 055 254
- WO-A1-2014/101943
- WO-A1-2014/101943
- US-A1- 2001 029 315
- US-A1- 2003 050 633
- US-A1- 2004 147 947
- US-A1- 2006 074 405
- US-A1- 2006 074 405
- US-A1- 2009 275 940
- US-A1- 2012 098 609
- US-A1- 2012 098 609
- US-A1- 2014 155 888
- US-A1- 2014 155 888
- US-A1- 2014 180 274

## Description

### BACKGROUND

The invention relates generally to a surgical system including a console and a replaceable component that is directly or indirectly attached to the console.

The invention further relates to detection of one or more states of electrical arrangement of the replaceable component and control of one or more therapeutic functions of the replaceable component based on the detected one or more states.

US 2006/0074405 A1 discloses a surgical system having a console comprising a therapy signal generator configured to generate a therapy signal and a controller configured to control the therapy signal generator. Furthermore, the system comprises a first component configured to be directly or indirectly detachably attached to the console, wherein the first component is configured to be driven by the therapy signal to therapeutically affect a biological tissue. The surgical system further comprises a sensor circuit arranged to the console, the sensor circuit comprising a reference circuit configured to provide a baseline signal based on the device state signal.

US 2009/0275940 A1 discloses a surgical tool system with a powered tool that includes a memory in which data describing the characteristics of the power signals that are to be sourced to the tool are stored. A control console based on the data in the tool memory sources the power signal to the tool. Both, the data signals written out from the tool memory and the power signals from the console are exchanged over common console terminals and common tool terminals. An isolation circuit in the tool prevents the applied power signals from the console from adversely affecting the tool memory.

EP 2 055 254 A2 discloses a power supply for electro surgery. A high frequency power supply for applying electrical energy to a target site on or within a patient's body is described. The power supply includes an electrical output driver and an output current sensor for detecting the current output from the electrical output driver. A power limiting device is coupled to the current sensor, wherein the power limiting device moves from an operating mode to a standby mode by reducing power from a first operating power level to a second standby power level on the output driver, when current output from the output current sensor exceeds a predetermined threshold level. The supply also includes a periodic detection device for continuously detecting current output in the standby mode without increasing the power to the first operating power level.

US 2014/0180274 A1 discloses a surgical operation system having a power supply apparatus that generates a high frequency signal by a first signal producing section, a converter that performs conversion into other energy by supply of the second signal, a surgical treatment instrument including a pair of electrodes, a cable that connects the surgical treatment instrument to the power supply apparatus, a measurement section that measures a high frequency voltage and a high frequency current for a living tissue to which the high frequency signal is supplied via the cable and the surgical treatment instrument. Furthermore, the surgical operation system comprises a calculation section that calculates an electrostatic capacity value of the cable and the surgical treatment instrument from the high frequency voltage and the high frequency current that are measured. A determination section determines whether or not the calculated electrostatic capacity value is smaller than a threshold value for detecting wire breakage, and a control section that performs output control of the high frequency signal on a basis of a determination result by the determination section.

It is an object of the invention to provide an enhanced surgical system. The invention is defined in appended independent claim 1. Further embodiments are defined in appended dependent claims.

### BRIEF SUMMARY

In accordance with the invention, a surgical system is provided. The surgical system comprises: a console comprising: an electrical isolation device; a therapy signal generator configured to generate a therapy signal; and a controller configured to control the therapy signal generator; a first component configured to be directly or indirectly detachably attached to the console, wherein the first component is configured to be driven by the therapy signal to therapeutically affect a biological tissue; a sensor circuit arranged to the console, the sensor circuit comprising: a device state signal generation circuit configured to generate a device state signal; and a reference circuit configured provide a baseline signal based on the device state signal; and a first target state identifying circuit arranged to the first component, wherein the first target state identifying circuit is configured to be selectively electrically connected in a first connected state to the sensor circuit, and wherein in the first connected state, the first target state identifying circuit is configured to provide a first target state identifying signal based on the device state signal; wherein the sensor circuit is configured to output a feedback signal based on the baseline signal and the first target state identifying signal through the electrical isolation device, and wherein the controller is configured to control the therapy signal generator based on the feedback signal.

### BRIEF DESCRIPTION OF THE DRAWING

These and other features, aspects, and advantages of the present invention will become better understood when the following detailed description is read with reference to the accompanying drawings in which like characters represent like parts throughout the drawings, wherein:
FIG. 1 is a diagram showing a surgical system in accordance with invention.
FIG. 2 is a diagram showing a modification of the surgical system illustrated in FIG. 1.
FIG. 3 is a diagram showing an example of the surgical system illustrated in FIG. 1 wherein a target state detection circuit and three target state identification circuits are implemented as an astable oscillator circuit.
FIG. 4 is a diagram showing an example of the surgical system illustrated in FIG. 1 wherein a target state detection circuit and three target state identification circuits are implemented as an astable oscillator circuit.
FIG. 5 is a diagram showing an example of the surgical system illustrated in FIG. 1 wherein a target state detection circuit and three target state identification circuits are implemented as an voltage sensor.

### DETAILED DESCRIPTION

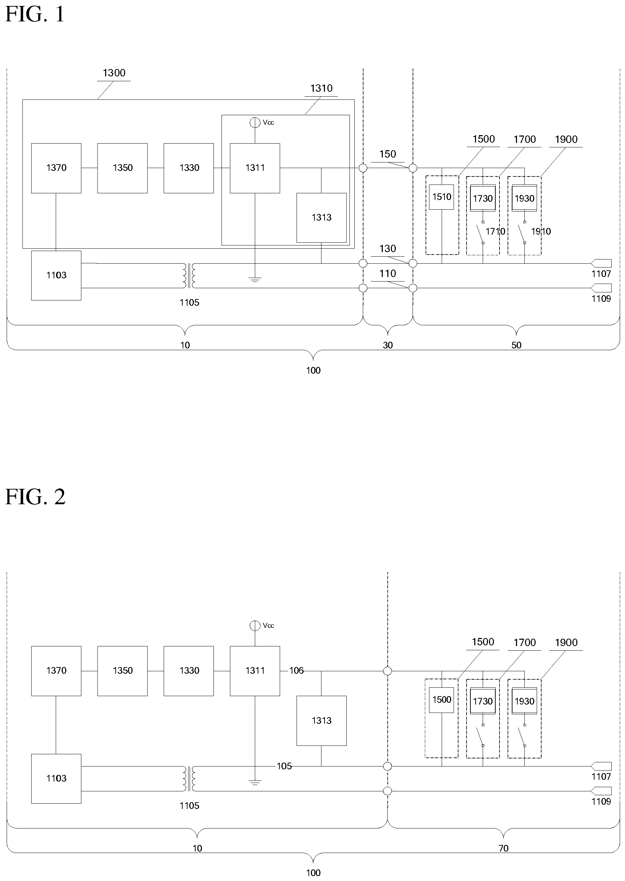

In the invention illustrated in FIG. 1, a surgical system 1 includes a console 10, an intermediate component 30 that is detachably attachable to the console 10, and a first component 50 that is detachably attachable to the intermediate component 30.

An example of the intermediate component 30 is a handpiece. An example of the first component 50 is an interchangeable tip that is configured to be detachably attached to the handpiece. Additional detailed descriptions of exemplary structures of the console 10, the intermediate component 30, and the first component 50 will be discussed further below.

The surgical system 1 further includes a circuit 100 that is distributively arranged to the console 10, the intermediate component 30, and the first component 50.

The circuit 100 includes an alternating current (AC) signal generator 1103, a step-up transformer 1105, a pair of electrical leads 1107, 1109, a target state detection circuit 1300 inlcuding a sensor circuit 1310, a first target state identification circuit 1500, and a second target state identification circuit 1700.

As illustrated in FIG. 1, the AC signal generator 1103, the step-up transformer 1105 and the target state detection circuit 1300 are arranged in the console 10, the first target state identification circuit 1500 and the second target state identification circuit 1700 are arranged in the first component 50, and the pair of electrical leads 1107, 1109 are arranged across the console 10, the intermediate component 30, and the first component 50.

The AC signal generator 1103 provides a time varying signal (and more specifically, an AC signal, to a low voltage side of the step-up transformer 1105. The step-up transformer 1105 steps up a voltage of the AC signal to a high voltage, and outputs the high voltage AC signal on a high voltage side of the step-up transformer 1105 to the pair of electrical leads 1107, 1109. The high voltage AC signal is characterized by at least one of a frequency and a voltage that is desirable for one or more therapeutic functions performed by the first component. Examples of the one or more therapeutic functions performed by the first component will be described in further detail below. The high voltage AC signal is referred to herein as a "therapy signal." The therapy signal may range, for example, from about 150 V to about 1500 V, with frequencies ranging from about 10 kHz to about 500 kHz.

In a modification of the surgical system 1, the AC signal generator 1103 and the step-up transformer 1105 can be replaced with a signal generator configured to generate a therapy signal that is characterized by at least one of a frequency and a voltage that is desirable for one or more therapeutic functions performed by the first component.

The sensor circuit 1310 is configured to detect one or more states of electrical arrangement of the first component 50 to the console 10 via the intermediate component 30. In a first arrangement, the first component 50 is not attached to the intermediate component 30. In a second arrangement, the first component 50 is attached to the console via the intermediate component 30. In a third arrangement, the first component 50 is attached to the console 10 via the intermediate component 30 and a switch provided to the first component 50 is actuated by a user.

The target state detection circuit 1300 includes the sensor circuit 1310. The sensor circuit 1310 includes a device state signal generation circuit 1311 and a reference circuit 1313. The device state signal generation circuit 1311 provides a device state signal that is referenced to one of the electrical leads 1107, 1109.

In the first arrangement, the reference circuit 1313 is electrically connected to the device state signal generation circuit 1311 to provide a baseline signal based on the device state signal.

In the second arrangement, the reference circuit 1313 and the first target state identification circuit 1500 are electrically connected to the device state signal generation circuit 1311. Specifically, the first target state identification circuit 1500 is electrically connected to the device state signal generation circuit 1311 upon physical attachment of the first component 50 to the intermediate component 30 and upon physical attachment of the intermediate component 30 to the console 10. In the second arrangement, the reference circuit 1313 and the first target state identification circuit 1500 provide the baseline signal and a first target state identification signal based on the device state signal.

In the third arrangement, the reference circuit 1313, the first target state identification circuit 1500, and the second target state identification circuit 1700 are electrically connected to the device state signal generation circuit 1311. The first target state identification circuit 1500 is electrically connected to the device state signal generation circuit 1311 upon physical attachment of the first component 50 to the intermediate component 30 and upon physical attachment of the intermediate component 30 to the console 10.

The second target state identification circuit 1700 includes a second target state identification circuit switch 1710 that is actuated by a user. The second target state identification circuit 1700 is electrically connected to the device state signal generation circuit 1311 upon physical attachment of the first component 50 to the intermediate component 30, physical attachment of the intermediate component 30 to the console 10 and actuation of the second target state identification circuit switch 1710 by the user.

In the third arrangement, the reference circuit 1313, the first target state identification circuit 1500, and the second target state identification circuit provide the baseline signal, the first target state identification signal, and a second target state identification signal based on the device state signal.

The target state detection circuit 1300 further includes an analog to digital convertor (ADC) 1330, an electrical isolation circuit 1350 and a controller 1370. The ADC 1330 is configured to convert a characteristic of the baseline signal, the first target state identifying signal and the second target state identifying signal to a digital signal. The digital signal is then passed through the electrical isolation circuit 1350 as a low voltage signal to the controller 1370 as feedback data. Examples of the electrical isolation circuit 1350 include an opto-isolator, a capacitive isolator, and an inductive isolator.

The controller 1370 is configured to control at least one therapeutic function performed by the first component 50 based on the feedback data.

In one example, the controller 1370 is implemented by hardware or a combination of hardware and software. The controller 1370 is configured to correlate the characteristic of the baseline signal, the first target state identification signal, and the second target state identification signal with one or more predetermined values in a look-up table. Based on the one or more predetermined values, the controller 1370 is configured to determine, for example, one or more of the following states of electrical arrangement of the first component 50: the first component 50 is not attached to the console 10, the first component 50 is attached to the console 10 and is a recognized component or one of a set of recognized components, the first component 50 is attached to the console 10 and is not a recognized component, a switch of a recognized component is activated, and the switch of the recognized component is not activated. The controller 1370 is further configured to control one or more therapeutic functions performed by the first component 50 based on the determined one or more states of electrical arrangement of the first component 50.

The surgical system 1 is not limited to a first target state identification circuit 1500 and a first target state identification circuit 1700. The surgical system 1 can include a third target state identification circuit 1900 and additional target state identification circuits that can be arranged to be electrical connected to the device state signal generation circuit. The third target state identification circuits can include a third target state switch 1910 that is actuated by the user to electrically connect the third target state identification circuit to the device state signal generation circuit 1311. The third and additional target state identification circuit are configured to provide a third and additional target state identification signals, the characteristics of which can be correlated by the controller 1370 with predetermined values in the look-up table. Based on the predetermined values, the controller 1370 is configured to determine additional states of electrical arrangement of the first component 50. The controller is configured to control additional therapeutic functions performed by the first component based on the determined additional states of electrical arrangement of the first component 50.

Examples of the sensor circuit 1310, the first target state identification circuit 1500 and the first target state identification circuit 1700 are discussed below.

The sensor circuit 1310, the first target state identification circuit 1500, the first target state identification circuit 1700, and the third target state identification circuit 1900 can be implemented by, for example, an astable oscillator circuit (or more generally, a capacitive sensing circuit).

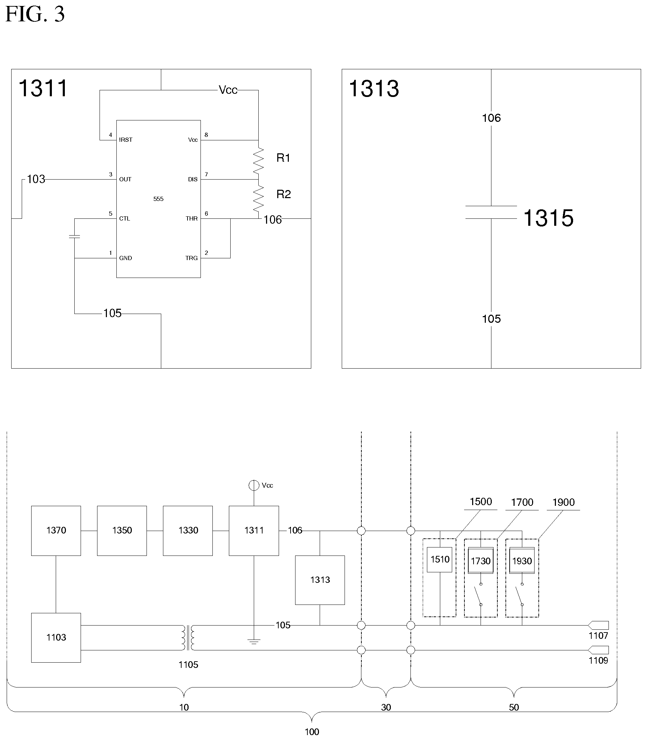

FIG. 3 illustrates an example of an astable oscillator circuit implemented with a 555 timer integrated circuit. The frequency of the astable oscillator output signal is controlled by the fixed electrical connection to the reference circuit 1313 (including the reference capacitor 1315) and (i) the selective electrical connection of the first target state identification circuit 1500 (including a first target state capacitor 1510) upon physical attachment of the first component 50 to the console 10 via the intermediate component 30, (ii) the selective electrical connection of the second target state identification circuit 1700 (including a second target state capacitor 1730 and a second target state switch) upon the actuation of the second target state switch 1710 by the user, and (iii) the selective electrical connection of the third target state identification circuit 1900 (including a third target state capacitor 1930 and a third target state switch 1910) upon the actuation of the third target state switch 1910 by the user. The astable oscillator's output signal frequency is indirectly proportional to the capacitance offered by the reference capacitor, the first target capacitor 1510, the second target capacitor 1730 and the third target capacitor 1930.

In FIG. 3, the trigger pin (2) and the threshold pin (6) are connected so as to form a self-trigger, causing the 555 timer IC to operate as an astable oscillator. Here resistor R1 and resistor R2 act as timing resistors and the discharge pin (7) is connected to the junction of resistor R1 and resistor R2. When the supply Vcc is connected, the reference capacitor 1315, and the selectively electrically connected first target capacitor 1510, second target capacitor 1730, and third target capacitor 1930 act like a timing capacitor and change toward Vcc. When the one or more capacitors get charged, the output pin (3) is held high. When the one or more capacitors voltage is just greater than (2/3) Vcc, the upper comparator of the 555 timer IC triggers the internal control flip flop and the one or more capacitors discharge towards the ground through resistor R2. During this discharge cycle the output is held low. During this discharge, as the voltage across the one or more capacitors reaches (1/3) Vcc the lower comparator is triggered and again it starts charging and the output is held high.

As additional capacitors are electrically connected, the offered capacitance increases, thereby decreasing the frequency of the astable oscillator output. Based on the predetermined ratings of the reference capacitor, the first target capacitor, the second target capacitor, and the third target capacitor, the frequency of the astable oscillator output can be correlated utilized to determine one or more states of electrical arrangement of the first component.

The output of the astable oscillator from pin 3 is referred to herein as a feedback signal. The feedback signal can be a square wave. In the above-described first arrangement where the first component is not attached to the console, the reference capacitor 1315 is selected such that the astable oscillator provides a feedback signal having a measurable baseline frequency. In the above-described second arrangement where the first component 50 is attached to the console 10, the first target state capacitor 1510 is selected such that the astable oscillator provides a feedback signal having a measurable first frequency that is different from the baseline frequency. In the above-described third arrangement where the first component 50 is attached to the console 10 and the second target switch 1710 is actuated to electrically connect the second target state capacitor 1730, the second target state capacitor 1730 is selected such that the astable oscillator provides a feedback signal having a measurable second frequency that is different from the baseline frequency and the first frequency. The controller 1370 is then configured to determine the capacitance provided in the astable oscillator by, for example, counting the pulses of the square wave in a predetermined time period. The determined capacitance then indicates the state of electrical arrangement of the first component 50 to the console 10. It is noted that even in the absence of an attachment of the first component 50 to the console 10 (i.e. the above-described first arrangement), the surgical system 1 and in particular the circuit 100 provides for the reference circuit 1313 including the reference capacitor 1315 in the console 10 such that the feedback signal based on the baseline frequency is provided to the controller 1370 to control the AC signal generator 1103.

In a modification of the circuit 100 shown in FIG. 3, the astable oscillator circuit configured using a 555 timer IC can be implemented by replacing the reference capacitor 1315 with a reference resistor, and replacing the one or more of the first target capacitor 1510, the second target capacitor 1730, and the third target capacitor with a corresponding one or more of a first target resistor, a second target resistor, and a third target resistor.

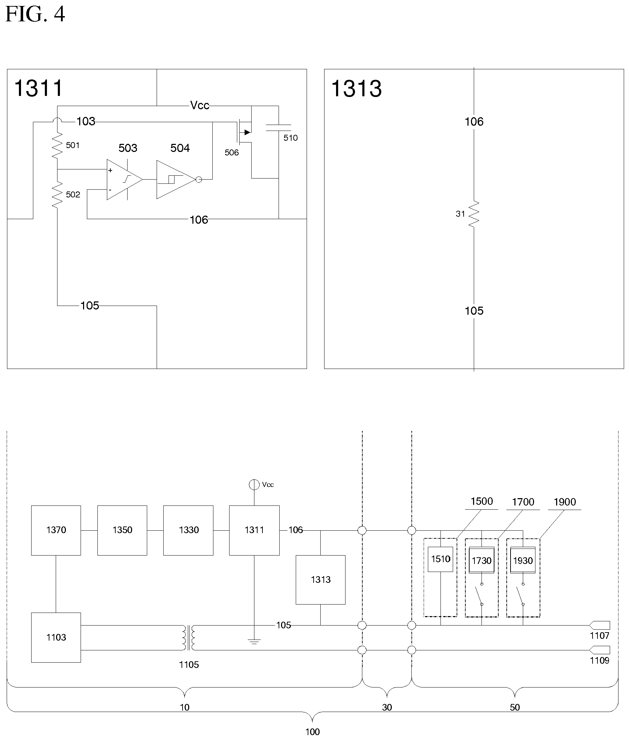

FIG. 4 illustrates another example of an astable oscillator circuit and a reference circuit that implements the sensor circuit 1310.

FIG. 4 shows an astable oscillator as a relaxation oscillator. A comparator takes in and compares a reference voltage from a voltage divider and the device state signal, and sends the output to a resetting latch which provides feedback to the comparator to provide the feedback signal to the controller. The resetting latch is comprised of a Schmidt trigger to provide hysteresis and a transistor switch to reset the oscillation.

In FIG. 4, a reference resistor 1315, a first target resistor 1510, a second target resistor 1730, and a third target resistor 1930 replaces the reference capacitor 1315, the first target capacitor 1510, the second target capacitor 1730, and the third target capacitor 1930 respectively described above with respect to FIG. 3.

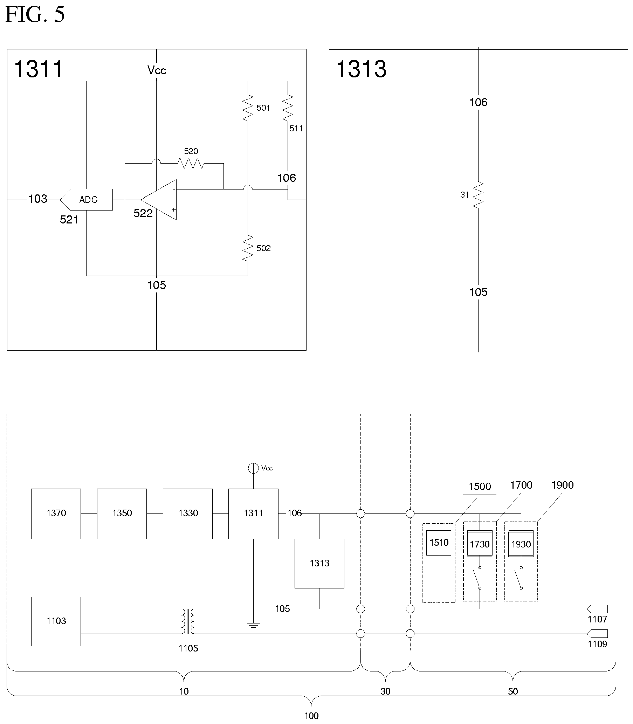

The sensor circuit 1310, the first target state identification circuit 1500, the first target state identification circuit 1700, and the third target state identification circuit 1900 can also be implemented by, for example, an a voltage sensor as illustrated in FIG. 5.

Figure 5 shows a device state signal sensing circuit for DC, non-time varying signals. A supply voltage is supplied to the reference resistor 1510 and the one or more target resistors by the supply Vcc through a pull up resistor 511 on bus 106. The device state signal voltage on bus 106 is compared by device 522 to a reference voltage supplied by the voltage divider comprised of resistors 501 and 502. Stability of the comparison is provided by a feedback resistor 520. The output of the comparator 522 is provided to an analog to digital converter 521 to create the feedback signal.

A more detailed description of the exemplary structures of the surgical system 1 will be discussed below. Examples of interchangeable tips that make up the first component and a handpiece that makes up the intermediate will be described below.

In a first example, a handpiece and a debrider blade as an interchangeable tip that is detachably attachable to the handpiece are provided. Detailed descriptions of the structures of the handpiece and the debrider blade can be found at, for example, U.S. Patent Application No. 13/803,380, published as US 2014-0155888 A1 on June 5th 2014.

The debrider blade can include a first (outer) tubular body and a second (inner) tubular body. The second tubular body is at least partially disposed within the first tubular body, and is arranged to be moved by rotation or reciprocation with respect to the first tubular body.

In the first example, a motor (or more generally, a power transfer device) that is suitable to move the second tubular body to rotate or reciprocate is arranged in the handpiece or in the interchangeable tip. The motor is in turn driven by a signal generator arranged in the console.

The first tubular body can include a first set of teeth arranged at an opening of the first tubular body. The second tubular body can include a second set of teeth arranged at an opening of the second tubular body. As the second tubular body is moved by rotation or reciprocation, a tissue that is arranged between the first set of teeth and the second set of teeth is sheared or dissected by the rotational or reciprocal movement of the second set of teeth.

As an alternative to the first set of teeth and the second set of teeth, the first tubular body can be provided with a first opening and the second tubular body can be replaced with a rotatable burr that can be driven by the motor to rotate to remove a target tissue.

In the first example, the interchangeable tip is provided with the first target state identification circuit 1500 and the first target state identification circuit 1700 described above.

A second example of the interchangeable tip includes a modification of the first example of the interchangeable tip. In the second example, bipolar cauterization and/or coagulation functions are provided. Specifically, the first tubular body is charged with a first electrical potential (as an active electrode) and the second tubular body is charged with a second electrical potential (as a return electrode) that is different for the first electrical potential. The active electrode is electrically connected to the electrical lead 1107 and the return electrode is electrically connected to the electrical lead 1109.

Referring back to FIG. 1, the interchangeable tip according to the second example is electrically connected to the handpiece through three pins 110, 130 and 150. The therapy signal is provided into the interchangeable tip through pins 110, 130. The first through third target state identifying signal are provided from the interchangeable tip to the sensor circuit 110 through pins 130 and 150. In the target state detection circuit 1300, the electrical isolation device 1350 then separates the feedback signal output by the sensor circuit 1310 from the therapy signal and transfers the feedback signal to the controller 1370.

As an alternative to the bipolar configuration described above, a monopolar configuration can be provided. In the monopolar configuration, the first tubular body is charged with a first electrical potential (as an active electrode) and a return electrode (as a second component) separate from the interchangeable tip is provided.

In the second example, the interchangeable tip is provided with the first target state identification circuit 1500 and the second target state identification circuit 1700, as described above, and a third target state identification circuit 1900 including a third target state identification switch 1910.

In an arrangement in which the third target state identification switch is actuated by the user, the controller may be configured to determine that the user has instructed a cauterization and/or coagulation function. Based on this determination, the controller 1370 may be configured to control the AC signal generator 1103 to enable cauterization and/or coagulation function.

The invention is not limited to the above-described examples. The invention also encompasses structures that can be driven to provide different combinations of the dissection, suction, and cauterization and/or coagulation functions described above.

The invention also encompasses, for example, an interchangeable tip that is configured to be controlled to perform other therapeutic functions such as dissection through ultrasonic vibrations. To enable dissection through ultrasonic vibrations, the first component can include a piezoelectric transducer and an end effector that is movably connected to the piezoelectric transducer. The piezoelectric transducer is electrically connected to electrical leads 1107, 1109 to be driven by the AC signal generator 1103.

A modification of the above-described surgical system 1 is illustrated in FIG. 2. According to the modification of the surgical system 1, the above-described indirect attachment of the first component 50 to the console 10 via the intermediate component 30 is replaced by a direct attachment of a first component 70 to the console 10. Specifically, the first target state identification circuit 1500 and the second target state identification circuit 1700 are electrically connected to the device state signal generation circuit 1311 upon physical attachment of the first component 50 to the console 10.

In another example, not covered by the claimed invention, a method for detecting one or more states of electrical arrangement of a replaceable component and control of one or more therapeutic functions of the replaceable component based on the detected one or more states is provided.

The method includes a step of providing a console including the step-up transformer 1105, the AC signal generator 1103 (or more generally, a therapy signal generator) configured to generate a therapy signal through the step-up transformer 1105, and a controller 1370 configured to control the AC signal generator 1103 (or therapy signal generator). The method further includes a step of providing a first component 50 configured to be directly or indirectly detachably attached to the console 10, wherein the first component 50 is configured to be driven by the therapy signal to therapeutically affect a biological tissue. The method further includes a step of providing a sensor circuit 1310 arranged to the console 10, the sensor circuit including a device state signal generation circuit 1311 configured to generate a device state signal, and a reference circuit 1313 configured output a baseline signal based on the device state signal. The method further includes providing a first target state identification circuit 1500 arranged to the first component 50, wherein the first target state identification circuit 1500 is configured to be selectively electrically connected in a first connected state to the sensor circuit, and wherein in the first connected state, the first target state identification circuit 1500 is configured to output a first target state identification signal based on the device state signal. The method further includes a step of enabling the sensor circuit 1310 to output the baseline signal and the first target state identification signal through an electrical isolation circuit 1350 as feedback data, and a step of enabling the controller 1370 to control the AC signal generator 1103 (or the therapy signal generator) based on the feedback data. The method further includes providing a first target state identification circuit 1700 to the first component 50. The method further includes enabling the second target state identification circuit 1700 to be selectively electrically connected to the sensor circuit 1310 by the actuation of a second target state switch 1710, wherein the second target state identification circuit 1700 outputs a second target state identification signal based on the device state signal. The method further includes enabling sensor circuit 1310 to output the second target state identification signal through the electrical isolation circuit 1350 as feedback data, and a step of enabling the controller 1370 to control the AC signal generator 1103 (or the therapy signal generator) based on the feedback data. The method further includes enabling the controller 1370 to continuously or periodically control the AC signal generator 1103 (or the therapy signal generator) based on the feedback data.

This written description uses examples to disclose the invention, including the best mode, and also to enable any person skilled in the art to practice the invention, including making and using any devices or systems. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Furthermore, to the extent that the terms "includes," "has" or "having" or variations in form thereof are used in either the detailed description or the claims, such terms are intended to be inclusive in a manner similar to the term "comprising" as "comprising" is interpreted when employed as a transitional word in a claim.

## Claims

1. A surgical system (1) comprising:
a console (10) comprising:
an electrical isolation device (1350);
a therapy signal generator (1103) configured to generate a therapy signal; and
a controller (1370) configured to control the therapy signal generator (1103);
a first component (50) configured to be directly or indirectly detachably attached to the console (10), wherein the first component (50) is configured to be driven by the therapy signal to therapeutically affect a biological tissue;
a sensor circuit (1310) arranged to the console (10), the sensor circuit (1310) comprising:
a device state signal generation circuit (1311) configured to generate a device state signal; and
a first target state identifying circuit (1500) arranged to the first component (50),
wherein the first target state identifying circuit (1500) is configured to be selectively electrically connected in a first connected state to the sensor circuit (1310), and
wherein in the first connected state, the first target state identifying circuit (1500) is configured to provide a first target state identifying signal based on the device state signal;
**characterized in that**
the console (10) comprises a reference circuit (1313) configured to provide a baseline signal based on the device state signal;
wherein the sensor circuit (1310) is configured to output:
a feedback signal based on the baseline signal and the first target state identifying signal through the electrical isolation device (1350); and
the feedback signal based on the baseline signal in the absence of the first target state identifying signal, thereby indicating absence of an attachment of the first component (50) to the console (10), through the electrical isolation device (1350),
wherein the controller (1370) is configured to:
perform first control of the therapy signal generator (1103) in response to the feedback signal based on the baseline signal and the first target state identifying signal; and
perform second control of the therapy signal generator (1103) in response to the feedback signal based on the baseline signal in the absence of the first target state identifying signal.

2. The surgical system (1) according to claim 1,
wherein the therapy signal generator (1103) is configured to generate the therapy signal as an AC signal, and to output the therapy signal to a pair of electrical leads (1107, 1109), wherein the feedback signal is referenced to one of the pair of electrical leads (1107, 1109), and
wherein the electrical isolation device (1350) separates the feedback signal from the therapy signal and transfers the feedback signal to the controller (1370).

3. The surgical system (1) according to claim 1, wherein the sensor circuit (1310) is arranged as an astable oscillator circuit.

4. The surgical system (1) according to claim 1, wherein the sensor circuit (1310) and the first target state identifying circuit (1500) are arranged as an astable oscillator circuit.

5. The surgical system (1) according to claim 1, wherein the sensor circuit (1310) is arranged as a voltage sensor circuit.

6. The surgical system (1) according to claim 1, wherein the sensor circuit (1310) and the first target state identifying circuit (1500) are arranged as a voltage sensor circuit.

7. The surgical system (1) according to claim 1,
wherein the reference circuit (1313) comprises a reference capacitor (1315) or a reference resistor (1315), and
wherein the first target state identifying circuit (1500) comprises one of:
a first target capacitor (1510) configured, in the first connected state, to be selectively electrically connected in parallel with the reference capacitor (1315), and
a first target resistor (1510) configured, in the first connected state, to be selectively electrically connected in parallel with the reference resistor (1315).

8. The surgical system (1) according to claim 7, further comprising a second target state identifying circuit (1700) arranged to the first component (50),
wherein the second target state identifying circuit (1700) is configured to be selectively electrically connected in a second connected state to the sensor circuit (1310),
wherein the second target state identifying circuit (1700) comprises:
one of:
a second target capacitor (1730) configured, in the second connected state, to be selectively electrically connected in parallel with the reference capacitor (1315) and the first target capacitor (1510), and
a second target resistor (1730) configured, in the second connected state, to be selectively electrically connected in parallel with the reference resistor (1315) and the first target resistor (1510); and
a second target switch (1710) configured to be controlled to electrically connect the one of the second target capacitor (1730) and the second target resistor (1730) in parallel with the corresponding reference capacitor (1315) and reference resistor (1315),
wherein in the second connected state, the second target state identifying circuit (1700) is configured to provide a second target state identifying signal based on the device state signal, wherein the sensor circuit (1310) is further configured to output the feedback signal based on the baseline signal, the first target state identifying signal and the second target state identifying signal through the electrical isolation device (1315), and wherein the controller (1370) is configured to control the therapy signal generator (1103) based on the feedback signal.

9. The surgical system (1) according to claim 7, wherein the one of the first target capacitor (1510) and the first target resistor (1510) of the first target state identifying circuit (1500) is configured to be electrically connected in parallel with the corresponding reference capacitor (1315) and reference resistor (1315) by attaching the first component (50) to the console (10).

10. The surgical system (1) according to claim 1, wherein the first component (50) is configured to be indirectly attached to the console (10) via an intermediate component (30) that is configured to be detachably attachable to the console (10).

## Patentansprüche

1. Chirurgisches System (1), umfassend:
eine Konsole (10), umfassend:
eine elektrische Isolationsvorrichtung (1350);
einen Therapiesignalgenerator (1103), der so ausgestaltet ist, dass er ein Therapiesignal erzeugt; und
eine Steuerung (1370), die so ausgestaltet ist, dass sie den Therapiesignalgenerator (1103) steuert;
eine erste Komponente (50), die so ausgestaltet ist, dass sie direkt oder indirekt abnehmbar an der Konsole (10) befestigt ist, wobei die erste Komponente (50) so ausgestaltet ist, dass sie von einem Therapiesignal zum therapeutischen Beeinflusssen eines biologischen Gewebes betrieben wird;
eine Sensorschaltung (1310), die an der Konsole (10) angeordnet ist, wobei die Sensorschaltung (1310) umfasst:
eine Vorrichtungszustandssignal-Erzeugungsschaltung (1311), die so ausgestaltet ist, dass sie ein Vorrichtungszustandssignal erzeugt; und
eine erste Zielzustandserkennungsschaltung (1500), die an der ersten Komponente (50) angeordnet ist,
wobei die erste Zielzustandserkennungsschaltung (1500) so ausgestaltet ist, dass sie in einem ersten verbundenen Zustand selektiv elektrisch mit der Sensorschaltung (1310) verbunden ist, und
wobei die erste Zielzustandserkennungsschaltung (1500) so ausgestaltet ist, dass sie im ersten verbundenen Zustand ein erstes Zielzustandserkennungssignal basierend auf dem Vorrichtungszustandssignal bereitstellt;
**dadurch gekennzeichnet, dass**:
die Konsole (10) eine Referenzschaltung (1313) umfasst, die so ausgestaltet ist, dass sie ein Basissignal basierend auf dem Vorrichtungszustandssignal bereitstellt;
wobei die Sensorschaltung (1310) so ausgestaltet ist, dass sie ausgibt:
ein Rückkopplungssignal basierend auf dem Basissignal und dem ersten Zielzustandserkennungssignal durch die elektrische Isolationsvorrichtung (1350); und
das Rückkopplungssignal basierend auf dem Basissignal beim Nichtvorhandensein des ersten Zielzustandserkennungssignals, wodurch das Nichtvorhandensein einer Befestigung der ersten Komponente (50) an der Konsole (10) angegeben wird, durch die elektrische Isolationsvorrichtung (1350),
wobei die Steuerung (1370) ausgestaltet ist zum:
Durchführen erster Steuerung des Therapiesignalgenerators (1103) als Reaktion auf das Rückkopplungssignal basierend auf dem Basissignal und dem ersten Zielzustandserkennungssignal; und
Durchführen zweiter Steuerung des Therapiesignalgenerators (1103) als Reaktion auf das Rückkopplungssignal basierend auf dem Basissignal bei Nichtvorhandensein des ersten Zielzustandserkennungssignals.

2. Chirurgisches System (1) nach Anspruch 1,
wobei der Therapiesignalgenerator (1103) so ausgestaltet ist, dass er das Therapiesignal als ein Wechselstromsignal erzeugt und das Therapiesignal an ein Paar elektrische Leitungen (1107, 1109) ausgibt, wobei das Rückkopplungssignal auf eine von dem Paar elektrische Leitungen (1107, 1109) bezogen ist, und
wobei die elektrische Isolationsvorrichtung (1350) das Rückkopplungssignal von dem Therapiesignal trennt und das Rückkopplungssignal an die Steuerung (1370) überträgt.

3. Chirurgisches System (1) nach Anspruch 1, wobei die Sensorschaltung (1310) als eine instabile Oszillatorschaltung angeordnet ist.

4. Chirurgisches System (1) nach Anspruch 1, wobei die Sensorschaltung (1310) und die erste Zielzustandserkennungsschaltung (1500) als eine instabile Oszillatorschaltung angeordnet sind.

5. Chirurgisches System (1) nach Anspruch 1, wobei die Sensorschaltung (1310) als eine Spannungssensorschaltung angeordnet ist.

6. Chirurgisches System (1) nach Anspruch 1, wobei die Sensorschaltung (1310) und die erste Zielzustandserkennungsschaltung (1500) als eine Spannungssensorschaltung angeordnet sind.

7. Chirurgisches System (1) nach Anspruch 1,
wobei die Referenzschaltung (1313) einen Bezugskondensator (1315) oder einen Bezugswiderstand (1315) umfasst, und
wobei die erste Zielzustandserkennungsschaltung (1500) eines umfasst von:
einem ersten Zielkondensator (1510), der so ausgestaltet ist, dass er im ersten verbundenen Zustand selektiv elektrisch mit dem Bezugskondensator (1315) parallelgeschaltet ist, und
einem ersten Zielwiderstand (1510), der so ausgestaltet ist, dass er im ersten verbundenen Zustand selektiv elektrisch mit dem Bezugswiderstand (1315) parallelgeschaltet ist.

8. Chirurgisches System (1) nach Anspruch 7, das ferner eine zweite Zielzustandserkennungsschaltung (1700) umfasst, die an der ersten Komponente (50) angeordnet ist,
wobei die zweite Zielzustandserkennungsschaltung (1700) so ausgestaltet ist, dass sie in einem zweiten verbundenen Zustand selektiv elektrisch mit der Sensorschaltung (1310) verbunden ist,
wobei die zweite Zielzustandserkennungsschaltung (1700) umfasst:
eines von:
einem zweiten Zielkondensator (1730), der so ausgestaltet ist, dass er im zweiten verbundenen Zustand selektiv elektrisch mit dem Bezugskondensator (1315) und dem ersten Zielkondensator (1510) parallelgeschaltet ist, und
einem zweiten Zielwiderstand (1730), der so ausgestaltet ist, dass er im zweiten verbundenen Zustand selektiv elektrisch mit dem Bezugswiderstand (1315) und dem ersten Zielwiderstand (1510) parallelgeschaltet ist; und
einem zweiten Zielschalter (1710), der so ausgestaltet ist, dass er so gesteuert wird, dass er den einen des zweiten Zielkondensators (1730) und des zweiten Zielwiderstands (1730) elektrisch mit dem entsprechenden Bezugskondensator (1315) und Bezugswiderstand (1315) parallelschaltet,
wobei die zweite Zielerkennungsschaltung (1700) so ausgestaltet ist, dass sie im zweiten verbundenen Zustand ein zweites Zielzustandserkennungssignal basierend auf dem Vorrichtungszustandssignal bereitstellt, wobei die Sensorschaltung (1310) ferner so ausgestaltet ist, dass sie das Rückkopplungssignal basierend auf dem Basissignal, dem ersten Zielzustandserkennungssignal und dem zweiten Zielzustandserkennungssignal durch die elektrische Isolationsvorrichtung (1315) ausgibt, und
wobei die Steuerung (1370) so ausgestaltet ist, dass sie den therapeutischen Signalgenerator (1103) basierend auf dem Rückkopplungssignal steuert.

9. Chirurgisches System (1) nach Anspruch 7, wobei der eine des ersten Kondensators (1510) und des ersten Zielwiderstands (1510) der ersten Zielzustandserkennungsschaltung (1500) so ausgestaltet ist, dass er durch Befestigen der ersten Komponente (50) an der Konsole (10) elektrisch mit dem entsprechenden Bezugskondensator (1315) und Bezugswiderstand (1315) parallelgeschaltet ist.

10. Chirurgisches System (1) nach Anspruch 1, wobei die erste Komponente (50) so ausgestaltet ist, dass sie indirekt über eine Zwischenkomponente (30), die so ausgestaltet ist, dass sie abnehmbar an der Konsole (10) befestigbar ist, an der Konsole (10) befestigt ist.

## Revendications

1. Un système chirurgical (1) comprenant :
une console (10) comprenant :
un dispositif (1350) d'isolation électrique ;
un générateur (1103) de signal thérapeutique configuré pour générer un signal thérapeutique ; et
un contrôleur (1370) configuré pour commander le générateur (1103) de signal thérapeutique ;
un premier composant (50) configuré pour être directement ou indirectement fixé de manière amovible à la console (10), le premier composant (50) étant configuré de façon à être piloté par le signal thérapeutique afin d'affecter thérapeutiquement un tissu biologique ;
un circuit (1310) formant capteur agencé sur la console (10), le circuit formant capteur (1810) comprenant :
un circuit (1311) de génération de signal d'état de dispositif configuré pour générer un signal d'état de dispositif ; et
un premier circuit (1500) d'identification d'état cible agencé au niveau du premier composant (50),
le premier circuit (1500) d'identification d'état cible étant configuré pour être sélectivement connecté électriquement dans un premier état connecté au circuit (1310) formant capteur, et,
dans le premier état connecté, le premier circuit (1500) d'identification d'état cible étant configuré pour fournir un premier signal d'identification d'état cible sur la base du signal d'état de dispositif ;
**caractérisé en ce que**
la console (10) comprend un circuit de référence (1313) configuré pour fournir un signal de base en fonction du signal d'état du dispositif ;
le circuit (1310) formant capteur étant configuré pour délivrer :
un signal de retour basé sur le signal de base et le premier signal d'identification d'état cible via le dispositif (1350) d'isolation électrique ; et le signal de retour étant basé sur le signal de base en l'absence du premier signal d'identification d'état cible, indiquant ainsi l'absence d'une fixation du premier composant (50) à la console (10), via le dispositif (1350) d'isolation électrique,
le contrôleur (1370) étant configuré pour :
effectuer une première commande du générateur (1103) de signal thérapeutique en réponse au signal de retour sur la base du signal de base et du premier signal d'identification d'état cible ; et
effectuer une deuxième commande du générateur (1103) de signal thérapeutique en réponse au signal de retour sur la base du signal de base en l'absence du premier signal d'identification d'état cible.

2. Le système chirurgical (1) selon la revendication 1,
dans lequel le générateur (1103) de signal thérapeutique est configuré pour générer le signal thérapeutique en tant que signal en courant alternatif, et pour délivrer le signal thérapeutique à une paire de conducteurs électriques (1107, 1109),
dans lequel le signal de retour est référencé à un conducteur de la paire de conducteurs électriques (1107, 1109), et
dans lequel le dispositif (1350) d'isolation électrique sépare le signal de retour du signal thérapeutique et transfère le signal de retour au contrôleur (1370).

3. Le système chirurgical (1) selon la revendication 1, dans lequel le circuit (1310) formant capteur est agencé comme un circuit oscillateur astable.

4. Le système chirurgical (1) selon la revendication 1, dans lequel le circuit (1310) formant capteur et le premier circuit (1500) d'identification d'état cible sont agencés sous la forme d'un circuit oscillateur astable.

5. Le système chirurgical (1) selon la revendication 1, dans lequel le circuit (1310) formant capteur est agencé comme un circuit formant capteur de tension.

6. Le système chirurgical (1) selon la revendication 1, dans lequel le circuit (1310) formant capteur et le premier circuit (1500) d'identification d'état cible sont agencés sous la forme d'un circuit formant capteur de tension.

7. Le système chirurgical (1) selon la revendication 1,
dans lequel le circuit de référence (1313) comprend un condensateur de référence (1315) ou une résistance de référence (1315), et
dans lequel le premier circuit (1500) d'identification d'état cible comprend un parmi :
un premier condensateur cible (1510) configuré, dans le premier état connecté, pour être sélectivement connecté électriquement en parallèle avec le condensateur de référence (1315), et
une première résistance cible (1510) configurée, dans le premier état connecté, pour être sélectivement connectée électriquement en parallèle avec la résistance de référence (1315).

8. Le système chirurgical (1) selon la revendication 7, comprenant en outre un deuxième circuit (1700) d'identification d'état cible agencé au niveau du premier composant (50),
le deuxième circuit (1700) d'identification d'état cible étant configuré pour être sélectivement connecté électriquement dans un deuxième état connecté au circuit (1310) formant capteur,
le deuxième circuit (1700) d'identification d'état cible comprend :
un parmi :
un deuxième condensateur cible (1730) configuré, dans le deuxième état connecté, pour être sélectivement connecté électriquement en parallèle avec le condensateur de référence (1315) et le premier condensateur cible (1510), et
une deuxième résistance cible (1730) configurée, dans le deuxième état connecté, pour être sélectivement connecté électriquement en parallèle avec la résistance de référence (1315) et la première résistance cible (1510) ; et
un deuxième commutateur cible (1710) configuré pour être commandé de façon à connecter électriquement celui parmi le deuxième condensateur cible (1730) et la deuxième résistance cible (1730) en parallèle avec le condensateur de référence (1315) et la résistance de référence (1315) correspondants ;
dans le deuxième état connecté, le deuxième circuit (1700) d'identification d'état cible est configuré pour fournir un deuxième signal d'identification d'état cible sur la base du signal d'état de dispositif,
le circuit (1310) formant capteur étant en outre configuré pour délivrer le signal de retour sur la base du signal de base, du premier signal d'identification d'état cible et du deuxième signal d'identification d'état cible via le dispositif d'isolation électrique (1315), et
le contrôleur (1370) étant configuré pour commander le générateur (1103) de signal thérapeutique sur la base du signal de retour.

9. Le système chirurgical (1) selon la revendication 7, dans lequel l'un parmi le premier condensateur cible (1510) et la première résistance cible (1510) du premier circuit (1500) d'identification d'état cible est configuré pour être connecté électriquement en parallèle avec le condensateur de référence (1315) et la résistance de référence (1315) correspondants en fixant le premier composant (50) à la console (10).

10. Le système chirurgical (1) selon la revendication 1, dans lequel le premier composant (50) est configuré pour être fixé indirectement à la console (10) par l'intermédiaire d'un composant intermédiaire (30) qui est configuré pour pouvoir être fixé de manière amovible à la console (10).
